# EUROPÄISCHE PATENTSCHRIFT

(11) **EP 1 888 139 B1**
(45) Veröffentlichungstag und Bekanntmachung des Hinweises auf die Patenterteilung: **29.02.2012**
(21) Anmeldenummer: 06741621.4
(22) Anmeldetag: 01.06.2006
(51) Int. Cl.: A61L 27/56, A61L 27/10

(54) **DENTALIMPLANTAT UND VERFAHREN ZU DESSEN HERSTELLUNG**
DENTAL IMPLANT AND METHOD FOR THE PRODUCTION THEREOF
IMPLANT DENTAIRE ET PROCEDE DE FABRICATION

(30) Priorität: 06.06.2005 CH 947052005
(43) Veröffentlichungstag der Anmeldung: 20.02.2008
(73) Patentinhaber: THOMMEN MEDICAL AG, 4437 Waldenburg (CH)
(72) Erfinder: SCHLOTTIG, Falko, CH-4414 Füllinsdorf (CH); HEFTI, Thomas, CH-4056 Basel (CH)
(74) Vertreter: Bremi, Tobias Hans
(86) Internationale Anmeldenummer: PCT/CH2006/000296
(87) Internationale Veröffentlichungsnummer: WO 2006/131010

(56) Entgegenhaltungen:
- WO-A-01/32228
- DE-A1- 2 641 695
- DE-A1- 3 015 529
- DE-A1- 4 114 792
- DE-C1- 19 944 970
- JP-A- 61 201 642

## Beschreibung

### TECHNISCHES GEBIET

Die vorliegende Erfindung betrifft ein Implantat, insbesondere ein Dentalimplantat, mit einer porösen Oberfläche zum wenigstens teilweisen Einsetzen in einen Knochen, welches verbesserte Osteointegrationseigenschaften aufweist. Das Implantat ist dabei keramisch. Weiterhin betrifft die vorliegende Erfindung ein Verfahren zur Herstellung eines solchen Implantates sowie Verwendungen eines solchen Implantates.

### STAND DER TECHNIK

Verletzte oder beschädigte Teile des Hart- und/oder Weichgewebes des menschlichen Körpers werden am Besten wiederhergestellt, indem körpereigenes Hart- und/oder Weichgewebe verwendet wird. Dies ist aus verschiedenen Gründen nicht immer möglich, und daher kommt in vielen Fällen synthetisches Material als temporäres (bioabbaubares respektive postoperativ entfernbares) oder permanentes Ersatzmaterial zum Einsatz.

Implantate, welche im Hart- und/oder Weichgewebe verankert werden, dienen dem temporären oder permanenten Ersatz oder dem Support von unfall-, abnützungs-, mangelerscheinungs- oder krankheitsgeschädigten oder sonst degenerierten Teilen des Bewegungsapparates, einschliesslich insbesondere des Kauapparates. Als Implantat wird normalerweise ein künstliches, chemisch stabiles Material bezeichnet, welches als plastischer Ersatz oder zur mechanischen Verstärkung in den Körper eingebracht wird (vgl. z.B. Roche Lexikon Medizin, Urban & Fischer, (Hrsg.); 5. Aufl. 2003). Die Hilfs- und Ersatzfunktion im Körper wird auf Basis der mechanischen Eigenschaften und des Implantatdesigns übernommen. So sind beispielsweise Hüft- und Kniegelenksprothesen, Wirbelsäulenimplantate und Dentalimplantate seit vielen Jahren im erfolgreichen klinischen Einsatz.

Für die Implantatverankerung und die Implantatverträglichkeit an der Grenzfläche Implantatoberfläche / angrenzendes Gewebe hat die Implantatoberfläche eine grosse Bedeutung. So haben Messungen gezeigt, dass Implantate mit glatter Oberfläche beinahe unabhängig vom verwendeten Basismaterial nur wenig im Knochen verankert werden (schlechte Osteointegration), während Implantate mit einer strukturierten Oberfläche eine gute mechanische und bei entsprechender Gestaltung der Oberfläche auch eine gute biologische Verbindung mit dem umgebenden Hart- oder Weichgewebe eingehen (vgl. z.B. Titanium in Medicine, Material Science, Surface Science, Engineering, Biological Responses and Medical Applications Series: Engineering Materials, Brunette, D.M.; Tengvall, P.; Textor, M.; Thomsen, P. (Eds.)).

Die für ein genügendes Einwachsen benötigte Zeit, eine für Implantate wichtige und zentrale Eigenschaft, wird als Osteointegrationszeit, respektive im Dentalbereich auch als Osseointegrationszeit bezeichnet. Damit wird die Zeit beschrieben, die vergeht, bis sich die Knochensubstanz in genügender Stärke und dauerhaft mit der Implantatoberfläche verbunden hat, das heisst sich gewissennassen in diese integriert hat.

Zur Oberflächenbehandlung und Oberflächenstrukturierung werden verschiedenste Methoden verwendet, siehe z.B. A Guide to Metal and Plastic Finishing (Maroney, Marion L.; 1991); Handbook of Semiconductor Electrodeposition (Applied Physics, 5) (Pandey, R. K., et. al.; 1996); Surface Finishing Systems: Metal and Non-Metal Finishing Handbook-Guide (Rudzki, George J.; 1984); Titanium in Medicine, Material Science, Surface Science, Engineering, Biological Responses and Medical Applications Series: Engineering Materials, (Brunette, D.M.; Tengvall, P.; Textor, M.; Thomsen, P. (Eds.)) und Materials and Processes for Surface and Interface Engineering (NATO Asi Series. Series E, Applied Sciences, 115, Pauleau, Ives (Editor); 1995); und die darin genannten Referenzen.

Implantate werden heute aus verschiedensten Materialien hergestellt, wie beispielsweise aus Titan, Niob, Zirkon, Tantal, aus Legierungen wie z.B. Titanlegierungen, Implantatstahl, aus CoCr Legierungen, aus verschiedenen Polymeren und Keramiken z.B. auf Basis von Zirkonoxiden, Aluminiumoxiden, Titanoxiden etc..

Für viele Implantate, speziell für Zahnimplantate, werden hauptsächlich Titan und seine Legierungen verwendet, da diese Materialien ein ausreichend niedriges Elastizitätsmodul und eine relativ hohe Festigkeit aufweisen. Allerdings haben Messungen gezeigt, dass sich Titan-Implantate mit glatter Oberflächenstruktur nur ungenügend im Knochen verankern, während Implantate mit aufgerauter Oberfläche einen bezüglich der Zug- und Torsionsfestigkeit merklich verbesserten Knochen-Implantat-Verbund ergeben.

In der EP 0 388 576 A1 wird deshalb vorgeschlagen, auf einer metallischen Implantatoberfläche in einem ersten Schritt mittels Sandstrahlen eine Makrorauhigkeit aufzubringen und diese anschliessend mittels Behandlung in einem Säurebad mit einer Mikrorauhigkeit zu überlagern. So kann die Implantatoberfläche mittels Sandstrahlen aufgeraut und anschliessend mit einem Ätzmittel, z. B. Fluorwasserstoffsäure oder einem Chlorwasserstoffsäure/Schwefelsäuregemisch behandelt werden. Durch diese Strukturierung der Oberfläche wird eine sichere Verbindung zwischen Hartgewebe und Metall erreicht.

Im Bereich der Dentalimplantate ist Titan, insbesondere im vorderen, sichtbaren Oralbereich, aus ästhetischen Gründen ungeeignet, da sich das Material optisch von der Hart- und der sichtbaren Weichgewebeumgebung unterscheidet. Daher ist es erstrebenswert, einen anderen Werkstoff, der diese Nachteile nicht aufweist, einzusetzen. Mit keramischen Werkstoffen wie beispielsweise Zirkonoxid, Titanoxid oder Aluminiumoxid oder Mischungen hiervon stehen Materialien zur Verfügung, die eine äusserst hohe Festigkeit aufweisen, insbesondere, wenn die Formkörper heiss-isostatisch gepresst oder heiss-isostatisch nachverdichtet sind. Eine spezifische yttriumstabilisierte Zirkonoxidkeramik, die etwa 92,1-93,5 Gew.-% Zr0₂, 4,5-5,5 Gew.-% Y₂0₃ und 3,8-2,2 Gew.-% HfO₂ aufweist, ist beispielsweise aus der US 6,165,925 bekannt. Andere gängige Keramiken werden in der Einleitung der US 6,165,925 diskutiert.

Der Einsatz von Keramik, beispielsweise einer Zirkonoxidkeramik, einer Titanoxidkeramik oder einer Aluminiumoxidkeramik, als Material zur Herstellung eines im Hart- oder Weichgewebe verankerten Implantates ist aufwändig, da es für eine ausreichende mechanische Stabilität der Keramik notwendig ist, diese ohne messbare Porosität herzustellen, wobei sich i.d.R. gleichzeitig eine glatte, äusserst harte Oberfläche ergibt.

Für glatte Keramikoberflächen ist kein direkter und ausreichend mechanisch stabiler Verbund mit dem umgebenden Hartgewebe zu erwarten. Daher werden Implantate aus reinen Keramiken wie Zirkonoxid, Titanoxid oder Aluminiumoxid oder Mischungen hiervon bisher kaum im direkten Kontakt zu Hartgewebe verwendet. Zur Verankerung im Hartgewebe werden konstruktive Verbunde mit metallischen Implantatmaterialien eingesetzt, beispielsweise in der Hüftendoprothetik oder in der oralen Implantologie.

Beispielsweise wird in der DE 195 30 981 A1 eine präfabrizierte vollkeramische Implantatkonstruktion aus Zirkondioxid zum zahnfarbenen Aufbau implantatgetragener artifizieller Kronenstümpfe beschrieben. Das eigentliche Implantat besteht dabei aus oberflächenstrukturiertem metallischem Titan, die Ästhetik des sichtbaren Teiles wird über eine Zirkonoxidkeramik dargestellt.

In der WO 2004/096075 A1 wird ein Zahnimplantat aus einem einteiligen Grundkörper beschrieben, der aus Zirkonoxid oder aus einer Zirkonoxid/Aluminium Mischung besteht. Eine Oberflächenbehandlung ist nicht beschrieben, und es ist fraglich, ob eine solche Implantatstruktur überhaupt genügende Osseointegration zeigt.

Die FR 2 721 196 A1 beschreibt ein einteiliges, auf Zirkoniumoxid basierendes Implantat. Zur Verbesserung der Osteointegration soll der entsprechende Implantatteil mit einer Beschichtung, beispielsweise aus Hydroxyapatit, versehen werden.

In der WO03/045268 A1 wird ein Keramikimplantat auf der Basis von Zirkonoxid beschrieben. Die Aussenfläche des Verankerungsteils wird zumindest teilweise entweder mit einem abtragenden Verfahren aufgerauht oder mikrostrukturiert oder mit einer Beschichtung versehen. Dabei werden nach einer Strahlbehandlung, etwa durch Sandstrahlen, auch chemische Verfahren, insbesondere Ätzverfahren in Betracht gezogen, die teilweise ergänzend als Nachbehandlung zu einer vorherigen mechanischen Behandlung zur Anwendung kommen können. Bevorzugt ist insbesondere zunächst eine Strahlbehandlung, etwa durch Sandstrahlen mit Al₂0₃, und anschliessend eine Ätzbehandlung mit Phosphorsäure, Schwefelsäure, Salzsäure oder Mischungen hiervon. Weiterhin kann das behandelte Implantat in einer geeigneten Flüssigkeit, beispielsweise deionisiertem Wasser, oder in einer NaCl-Lösung gelagert werden. Auf diese Weise wird vermieden, dass die Oberfläche vor dem Einsetzen des Dentalimplantats durch Bestandteile der Luft ihre Aktivierung ganz oder teilweise verliert. So wird eine Osteointegration unterstützt.

Die Problematik dabei ist, dass mit einer solchen kombinierten Behandlung die Rautiefe infolge der hohen Härte der Zirkonoxidkeramik gering bleibt und dass die Keramik gegenüber der Behandlung mit Phosphorsäure, Schwefelsäure, Salzsäure oder Mischungen hiervon chemisch äusserst stabil ist.

Die WO 01/32228 beschreibt die Oberflächenmodifikation eines keramischen Implantates. Dabei wird ein keramisches Implantat zunächst mit einer starken sauren oder basischen wässrigen Lösung behandelt, um Hydroxid-Gruppen auf der Oberfläche zur Verfügung zu stellen. In einem anschließenden Schritt wird, um spezifische für die Knochenbildung wichtige Ionen auf resp. in die Oberfläche zu bringen, das Implantat in eine Salzschmelze insbesondere mit Calciumionen, Natriumionen, Kaliumionen oder Phosphationen eingetaucht. Angeblich verfügen derartige Implantate mit glatten Oberflächen über bessere Einheileigenschaften.

Ebenfalls die Behandlung eines keramischen Implantats mit einer Salzschmelze ist aus der DE 3015529 bekannt, auch hier wird die Salzschmelze dazu verwendet, spezifische Alkaliionen in die Oberfläche einzutragen respektive gegen die vorhandenen auszutauschen. Die Verwendung einer Salzschmelze zum polieren einer Oberfläche ist aus der DE 2641695 bekannt.

Die Erzeugung einer Kalziumphosphat Schicht auf der Oberfläche unter Verwendung einer Salzschmelze eines Kalziumnitrats ist für ein metallisches Implantat aus Titan oder einer Titanlegierung aus der DE 19944970 oder aus der JP 61201642 bekannt.

Im Zusammenhang mit Metalloberflächen von Implantaten ist aus der DE 4114792 die Verwendung von Salzschmelzen aus komplexen Metalloid-Fluoriden bekannt.

### DARSTELLUNG DER ERFINDUNG

Der Erfindung liegt somit die Aufgabe zugrunde, die Nachteile des Standes der Technik zu vermeiden und Implantate vorzuschlagen, welche im Hart- und Weichgewebe zügig und nachhaltig verankern und somit gute Osteointegration resp. Osseointegration zeigen. Konkret geht es also darum, ein verbessertes Implantat mit einer strukturierten, insbesondere porösen Oberfläche zum wenigstens teilweisen Einsetzen in Hartgewebe wie in einen Knochen und/oder in Weichgewebe vorzuschlagen, wobei das Implantat keramisch ist. Ferner soll ein geeignetes Herstellverfahren hierfür angegeben werden.

Die Lösung dieser Aufgabe wird dadurch erreicht, dass die strukturierte respektive poröse Oberfläche wenigstens bereichsweise salzschmelzenmodifiziert respektive das Resultat einer Salzschmelzenmodifikation ist.

Diese Aufgabe wird erfindungsgemäss also durch eine spezifisch behandelte und dadurch spezifische Eigenschaften aufweisende Oberfläche des Implantates nach Anspruch 1 gelöst, wobei die Behandlung sowohl über die gesamte Implantatoberfläche als auch von partiellen Anteilen der Implantatoberfläche erfolgen kann und unter Verwendung einer eine Salzschmelze aus Alkali- und/oder Erdalkali-Hydroxiden oder einer Mischung dieser Salze erfolgt.

Im Rahmen dieser Erfindung ist von Implantaten die Rede, welche auf keramischen Materialien beruhen.

Der Kern der Erfindung besteht somit darin, dass überraschenderweise festgestellt wurde, dass Implantate auf Basis von Keramik unter Verwendung einer Salzschmelze an der Oberfläche derart modifiziert werden können, dass sie anschliessend hervorragende Osteointegration resp. Osseointegration zeigen. Es zeigt sich, dass die Osteointegration resp. Osseointegration einer derart bearbeiteten Oberfläche besser ist, als die entsprechenden Werte für säuremodifizierte Oberflächen und/oder Oberflächen insbesondere von Keramiken, welche nur durch Sandstrahlen mit einer Makrorauhigkeit versehen wurden.

Das Implantat wird also an der Oberfläche durch eine Salzschmelze durch Ätzung strukturiert, wobei insbesondere bevorzugt bei der Ätzung in der Salzschmelze im wesentlichen ausschliesslich eine Abtragung von Material erfolgt. Es geht mit anderen Worten nicht darum, durch die Salzschmelze nur beispielsweise Anionen oder Kationen aus der Salzschmelze in die Oberfläche einzubringen oder gewissermassen eine Beschichtung zu bewirken, sondern es geht effektiv darum, durch die Salzschmelze einen die Topographie der Oberfläche verändernden Abtragungsprozess zu benutzen, um eine sehr spezifisch strukturierte Oberfläche zu erhalten.

Tatsächlich wird auch gefunden, dass die erfindungsgemäss hergestellten Oberflächen im wesentlichen keine eingetragenen Bestandteile der verwendeten Salzschmelze aufweisen. Die Salzschmelze wirkt also im wesentlichen rein abtragend.

Die entstehenden topologische Struktur entspricht dabei bei entsprechender Einstellung der Bedingungen und bei entsprechender Materialwahl des Implantats einer Mikrorauhigkeit, das heisst vorzugsweise einer Rauhigkeit mit einer Grössenordnung der Strukturelemente im oberen Nanometerbereich respektive unteren Mikrometerbereich (z.B. 100 nm - 5 µm, vorzugsweise 500mm - 2 µm, vergleiche auch Figuren), welche einer oben genannten, durch mechanische Behandlung bewirkten Makrorauigkeit (typischerweise > 10-20 µm) überlagert sein kann. Bevorzugtermassen handelt es sich bei der Oberflächenstruktur um eine derartige Mikrorauhigkeit mit einer blumenkohlartigen und/oder körnigen Oberflächentopologie.

Die Strukturierung der Oberfläche erfolgt bevorzugtermassen vollständig ohne jegliche zusätzliche Behandlung durch konzentrierte Säuren oder Basen oder wässrige Lösungen sondern nur durch die Beeinflussung durch die Salzschmelze, gegebenenfalls in Kombination mit einer mechanischen Behandlung zur Erzeugung der Makrorauigkeit. Zusätzliche Beschichtungen wie beispielsweise aus Apatit sind nicht erforderlich und bevorzugtermassen auch nicht vorhanden.

Als besonders geeignet erweist sich die salzschmelzenmodifizierte Oberfläche, wenn das Implantat wenigstens an der Oberfläche mit einer Schicht aus Keramik versehen ist, oder wenn, wie bevorzugt, das Implantat im wesentlichen vollständig aus Keramik besteht.

Bei der Keramik kann es sich um unterschiedliche Typen handeln, wobei diese aus dem Stand der Technik bekannt sind. Beispielsweise kann eine Keramik verwendet werden, welche Titanoxid oder Zirkonoxid enthält, welches gegebenenfalls zusätzlich mit Yttriumoxid und/oder Hafniumoxid, versetzt ist. Vergleiche dazu beispielsweise die US 6,165,925, deren Offenbarung hinsichtlich der Zusammensetzung und der Herstellung von solchen Keramiken auf Basis von Zirkonoxid ausdrücklich in den Offenbarungsgehalt der vorliegenden Beschreibung eingeschlossen werden soll.

Alternativ ist es möglich, Keramiken zu verwenden, welche Aluminiumoxid enthalten, welches gegebenenfalls zusätzlich mit Siliziumdioxid, Eisen(III)oxid und/oder Natriumoxid versetzt ist. Ebenfalls ist es möglich, eine Keramik zu verwenden, welche Siliziumnitrid enthält, welches gegebenenfalls zusätzlich mit Siliziumdioxid, Eisen(III)oxid und/oder Natriumoxid versetzt ist. Auch Keramiken basierend auf Mischungen oder Mehrschichtsystemen auf Basis der genannten Materialien sind möglich.

Gemäss einer bevorzugten Ausführungsform handelt es sich beim Implantat um ein Dentalimplantat, dessen im implantierten Zustand dem Knochen und/oder Weichgewebe ausgesetzte Oberfläche wenigstens bereichsweise salzschmelzenmodifiziert ist.

Die salzschmelzenmodifizierte Oberfläche kann überlagert sein mit einer Makrorauhigkeit, wie sie weiter oben beschrieben wurde, das heisst mit einer Makrorauhigkeit mit Porengrössen von mehr als 10 µm, vorzugsweise von mehr als 20 µm. Es kann sich dabei um eine sandstrahlmodifizierte Oberfläche handeln.

Des weiteren betrifft die vorliegende Erfindung ein Verfahren zur Herstellung eines Implantates, wie es oben beschrieben wird. Das Verfahren ist dadurch gekennzeichnet, dass ein Implantat aus Keramik gegebenenfalls nach vorgängiger abtragender Oberflächenmodifikation insbesondere zur Erzeugung einer Makrorauhigkeit, wenigstens in den Knochen und/oder Weichgewebe ausgesetzten Bereichen unter Zuhilfenahme einer Salzschmelze oberflächenmodifiziert wird. Typischerweise wird dazu ein Bad aus einer Salzschmelze verwendet.

Gemäss Erfindung handelt es sich bei der Salzschmelze um eine Salzschmelze aus Alkali- und/oder Erdalkali-Hydroxiden, oder einer Mischung dieser Salze.

Es handelt sich also bei der Salzschmelze um eine Salzschmelze mit wenigstens einem Hydroxid, konkret mit wenigstens einem Alkali- und/oder Erdalkali-Hydroxid. Bevorzugterweise handelt es sich bei der Salzschmelze um eine (eutektische) Salzschmelze ausschliesslich bestehend aus einem oder mehreren Hydroxiden, insbesondere aus einem oder mehreren Alkali- und/oder Erdalkali-Hydroxiden. Genau die Verwendung von wenigstens einem Alkali- und/oder Erdalkali-Hydroxid, bevorzugtermassen einer Mischung von solchen Hydroxiden, führt unerwarteter Weise zu einer optimalen Ausbildung der gewünschten Mikro-Rauigkeit, wie sie oben beschrieben wurde.

Die Mischungen können binär, ternär oder auch höher sein. Bevorzugtermassen wird eine Salzschmelze im wesentlichen aus Alkalihydroxiden, wie z.B. aus Kaliumhydroxid und/oder Natriumhydroxid und/oder Lithiumhydroxid verwendet. Geringe Bestandteile, typischerweise im Bereich von weniger als 5% oder sogar weniger als 2%, anderer Salze (nicht nur, aber bevorzugtermassen der oben genannten) oder anderer Additive, sei es zur Einstellung der Ätzwirkung oder zur Einstellung der Schmelzetemperatur, können zusätzlich vorhanden sein.

Als besonders geeignet erweisen sich beispielsweise binäre Salzschmelzen bevorzugt aus Kaliumhydroxid und Natriumhydroxid, wobei diese beiden Komponenten in einem Verhältnis von 2 : 1 bis 0.5 : 1, vorzugsweise im Bereich von 1.5 : 1 - 0.75 : 1 vorgelegt werden. Als ganz besonders geeignet erweist es sich, wenn das Verhältnis im Bereich von 1:1 1 oder 7:5 gewählt wird. Bei solchen binären Salzschmelzen insbesondere aus den genannten Komponenten wird bevorzugtermassen bei einer Temperatur im Bereich von 100-600 °C, insbesondere bei einer Temperatur im Bereich von 150-250 °C gearbeitet.

Als ebenfalls besonders geeignet erweisen sich beispielsweise ternäre Salzsclunelzen aus Kaliumhydroxid, Natriumhydroxid und Lithiumhydroxid, wobei diese drei Komponenten in einem Verhältnis im Bereich von 10-20 : 4-10 : 0.5-2, insbesondere im Bereich von 14 : 6 : 1 vorgelegt werden. Bei solchen ternären Salzschmelzen kann bevorzugtermassen bei einer Temperatur von 100 - 400°C gearbeitet werden, insbesondere bei einer Temperatur im Bereich von 150-250 °C.

Generell kann gesagt werden, dass typischerweise eine Salzschmelze bei einer Temperatur im Bereich von 80°C - 1300 °C verwendet werden kann, vorzugsweise im Bereich von 150°C - 600°C, insbesondere bei einer Temperatur im Bereich von 170-250 °C.

Gemäss einer weiteren bevorzugten Ausführungsform des erfindungsgemässen Verfahrens wird die Oberfläche wenigstens bereichsweise über eine Dauer von 10 Minuten bis 300 Stunden, vorzugsweise von 10 bis 100 Stunden, insbesondere von 25 bis 35 Stunden einer Salzschmelze, z. B. in Form eines Bades, ausgesetzt. Bevorzugtermassen wird eine Behandlungsdauer von wenigstens einer Stunde, weiter bevorzugt von wenigstens zwei Stunden, verwendet, um tatsächlich eine genügende Abtragung von Material des Implantats durch die Salzschmelze und die Bildung der erfindungsgemässen Mikro-Rauigkeit gewährleisten zu können. Gute Resultate für die Oberfläche lassen sich beispielsweise bei Verwendung von binären Mischungen respektive ternären Mischungen, wie sie oben angegeben sind, erreichen, wenn das Implantat während 30h einer Salzschmelze einer Temperatur von 100 - 210 °C ausgesetzt wird.

Es erweist sich als vorteilhaft, die Implantatoberfläche zumindest teilweise nach der Behandlung mit der Salzschmelze zu reinigen. Dies kann zum Beispiel im Ultraschall mit deionisiertem Wasser erfolgen, wobei nachfolgend in deionisiertem Wasser gewaschen und gespült wird.

Eine weitere bevorzugte Ausführungsform des Verfahrens zeichnet sich dadurch aus, dass die abtragende Oberflächenmodifikation insbesondere zur Erzeugung der Makrorauhigkeit durch eine Strahlbehandlung erfolgt, insbesondere durch Sandstrahlung. Eine solche Sandstrahlung geschieht vorzugsweise unter Verwendung von Aluminiumoxid-Partikeln mit einer durchschnittlichen Korngrösse von 0.05 - 0.25 mm oder 0.25 - 0.5 mm. Dabei wird bevorzugt mit einem Druck zwischen 1-10 bar, vorzugsweise zwischen 1 - 6 bar, insbesondere bevorzugt zwischen 2 - 5 bar gearbeitet. Im speziellen wird bei einem Druck von im Bereich von 4 bar gearbeitet.

Weiterhin betrifft die vorliegende Erfindung ein Implantat herstellbar oder hergestellt nach einem Verfahren, wie es oben beschrieben wurde.

Weitere bevorzugte Ausführungsformen der Erfindung sind in den abhängigen Ansprüchen beschrieben.

### KURZE ERLÄUTERUNG DER FIGUREN

Die Erfindung soll nachfolgend anhand von Ausführungsbeispielen im Zusammenhang mit den Abbildungen näher erläutert werden. Es zeigen:
- Fig. 1 und 2: SEM Aufnahmen in unterschiedlicher Auflösung zum weiter unten genannten Beispiel 1; und
- Fig. 3 und 4: SEM Aufnahmen in unterschiedlicher Auflösung zum weiter unten genannten Beispiel 2; und
- Fig. 5: die Resultate von mit zwei verschiedenen Material- und Oberflächenimplantattypen durchgeführten Versuchen.

### WEGE ZUR AUSFÜHRUNG DER ERFINDUNG

Die vorliegende Erfindung beschreibt die Möglichkeit, die Oberfläche von Implantaten, die aus keramischen Materialien gefertigt werden, zu strukturieren. Zweck der Oberflächenstrukturierung sind eine bessere Verankerung der Implantate im Hartgewebe, eine verbesserter Verbund zwischen Hartgewebe und Implantatoberfläche, ein besserer Verbund zwischen Weichgewebe und Implantatoberfläche und eine verbesserte Interaktion der Implantatoberfläche an der Grenzfläche Implantatoberfläche Hartgewebe und/oder Weichgewebe.

Die Herstellung der Zirkonoxid- Titanoxid- und/oder Aluminiumoxid und/oder Mischkeramik für Implantate ist grundsätzlich im Stand der Technik bekannt und soll entsprechend nicht weiter ausgeführt werden. In diesem Zusammenhang sei beispielhaft auf die Offenbarung des bereits eingangs genannten Dokumentes, das heisst der US 6,165,925, Bezug genommen.

Vorzugsweise bezieht sich die Erfindung auf Implantate, welche im Hart- und/oder Weichgewebe verankert werden und dem temporären oder permanenten Ersatz oder Support von unfall-, abnutzungs-, mangelerscheinungs- oder krankheitsgeschädigten oder anderswie degenerierten Teilen des Bewegungsapparates unter Einschluss des Kauapparates, insbesondere des Dentalbereiches mit den zugehörigen auch ästhetischen Aspekten, dienen. So sind beispielsweise Hüft- und Kniegelenksprothesen, Wirbelsäulenimplantate und Dentalimplantate seit vielen Jahren im klinischen Einsatz.

Die Aufgabe der verbesserten Osteointegrationseigenschaften respektive Osseointegrationseigenschaften wird erfindungsgemäss durch eine entsprechende Oberflächenstruktur respektive Oberflächenbehandlung der (Keramik-)Oberfläche des Implantates gelöst, wobei die Behandlung sowohl über die gesamte Implantatoberfläche als auch von partiellen Bereichen der Implantatoberfläche erfolgen kann. Durch eine derartige Oberflächenstrukturierung wird gewährleistet, dass die ansonsten bioinerten Keramiken, wie vorzugsweise Zirkonoxid, Titanoxid oder Aluminiumoxid oder Mischungen davon, in das Hart- und /oder Weichgewebe integriert werden können.

Die strukturelle und funktionelle Verankerung, z. B. eines Zahnimplantats, im Knochen wird in der Regel durch Anbringen einer Makrorauhigkeit, und/oder einer, gegebenenfalls zusätzlichen, Mikrorauhigkeit, erreicht. Die Makrorauhigkeit kann beispielsweise mit einem mechanischen Strahlprozess, die Mikrorauhigkeit nachfolgend beispielsweise entweder in einem additiven Prozess mittels Plasmatechnik, oder in einem subtraktiven Prozess durch chemische Ätzung auf der Oberfläche bewirkt werden. Wie fest das Implantat im Knochen verankert ist, kann mit mechanischen Messungen festgestellt werden. Zahlreiche Untersuchungen haben gezeigt, dass die genügende Verankerung eines Implantat im Knochen in hohem Mass von der Oberflächenbeschaffenheit des Implantats, insbesondere von der Rauhigkeit an dessen Oberfläche, abhängt.

Die vorliegende Erfindung beschreibt eine spezifische und neuartig erzeugte Rauhigkeit für eine vergrösserte wirksame Oberfläche zur besseren Osteointegration von Implantaten, welche aus Keramiken, vorzugsweise aus Titanoxid, Zirkonoxid oder Aluminiumoxid oder Mischungen davon hergestellt sind. Diese erfindungsgemäss biologisch wirksame Oberfläche kann durch Verwendung einer Salzschmelze ggf. in Kombination beispielsweise mit zusätzlicher mechanischer Bearbeitung und Strukturierung, Kugelstrahlen, Sandstrahlen und/oder anschliessender oder vorgängiger chemischer Behandlung, beispielsweise Ätzung mit Säure o.ä. oder durch eine Kombination solcher Verfahren hergestellt werden.

Die erfindungsgemässe Oberfläche kann beispielsweise hergestellt werden, indem man die Oberfläche mit der gewünschten Rauhigkeit bzw. Textur versieht. Insbesondere kann man das Implantat herstellen, indem man die Implantatoberfläche kugelstrahlt, sandstrahlt und/oder unter Verwendung von Plasmatechnik strukturiert, und anschliessend die Oberfläche mit einem chemischen Prozess mit einer Salzschmelze behandelt, bis eine entsprechende Oberflächenstrukturierung entstanden ist.

Wie erwähnt ätzt man das Implantat mit einer Salzschmelze. Salzschmelzen sind definitionsgemäss sehr vielseitige Flüssigkeiten aus geschmolzenen Salzen, welche aber im vorliegenden Zusammenhang noch nie Verwendung gefunden haben. Klassische Salzschmelzen haben eine Temperatur von 150°C bis 1300°C. In neuster Zeit werden auch niedrig schmelzende Salze in Salzschmelzen unter 80°C eingesetzt. Es zeigt sich überraschenderweise, dass insbesondere bei Implantaten auf Basis von Keramik Salzschmelzen eine für die Integration in Knochen respektive Weichgewebe hervorragende Ätzwirkung der Oberfläche haben, während die üblicherweise verwendeten Säurebäder Keramik nicht genügend angreifen können.

Vorzugsweise wird die Oberfläche in der vorliegenden Anwendung mit einem Gemisch von Kaliumhydroxid und Natriumhydroxid geätzt oder die Oberfläche wird mit einem Gemisch von Kaliumhydroxid, Natriumhydroxid und Lithiumhydroxid geätzt. Es ist aber neben diesen binären respektive ternären Mischungen auch möglich, Salzschmelzen auf Basis eines einzigen Salzes zu verwenden. Anschliessend wird die Oberfläche im Ultraschall mit reinem/deionisiertem Wasser behandelt und danach mit reinem/deionisiertem Wasser gespült und gewaschen.

Vorzugsweise wird mit einem Gemisch von Kaliumhydroxid und Natriumhydroxid im Verhältnis von 1:1 gearbeitet oder aber die Oberfläche wird mit einem Gemisch von Kaliumhydroxid, Natriumhydroxid und Lithiumhydroxid von etwa 14 : 6 : 1 geätzt. Vorzugsweise geht man so vor, dass man das Implantat sandstrahlt und anschliessend in einer Salzschmelze mit einem Gemisch von Kaliumhydroxid und Natriumhydroxid im Verhältnis von 1:1 oder aber mit einem Gemisch von Kaliumhydroxid, Natriumhydroxid und Lithiumhydroxid von etwa 14 : 6 : 1 bei 150 - 400 °C ätzt. Anschliessend wird die Oberfläche im Ultraschall mit reinem/deionisiertem Wasser behandelt und mit reinem/deionisiertem Wasser gespült und gewaschen.

Man kann das Implantat z. B. mit Aluminiumoxid Partikeln mit einer durchschnittlichen Korngrösse von 0.05-0.25 mm oder 0.25 - 0.5 mm sandstrahlen und anschliessend in einer Salzschmelze mit einem Gemisch von Kaliumhydroxid und Natriumhydroxid im Verhältnis von 1:1 oder aber mit einem Gemisch von Kaliumhydroxid, Natriumhydroxid und Lithiumhydroxid von etwa 14 : 6 : 1 bei 150 - 400 °C ätzen.

Die Strahlbehandlung kann hierbei etwa mit einem Druck zwischen etwa 1 bar und 10 bar, vorzugsweise zwischen 1 und 6 bar, insbesondere zwischen 2 und 5 bar durchgeführt werden.

Dabei wird insbesondere eine Ätzbehandlung mit einem Gemisch von Kaliumhydroxid, Natriumhydroxid und Lithiumhydroxid von etwa 14 : 6 : 1 bei 150 - 400 °C als Nachbehandlung zu einer Strahlbehandlung bevorzugt.

Die Ätzung in der Salzschmelze kann beispielsweise über eine Dauer von 10 Minuten bis 300 Stunden, vorzugsweise von 10 bis 100 Stunden, insbesondere von etwa 25 bis 35 Stunden durchgeführt werden.

Die Ätzung in der Salzschmelze kann bei einer Temperatur von 150°C bis 400°C , vorzugsweise bei 180°C bis 220°C, insbesondere von etwa 190°C bis 210°C durchgeführt werden.

An eine Ätzbehandlung in der Salzschmelze schliesst sich zweckmässigerweise ein Reinigungsschritt an, etwa im Ultraschall mit reinem/deionisiertem Wasser und durch Waschen in deionisiertem Wasser und anschliessendem Spülen in deionisiertem Wasser.

Mit derartig vorbehandelten Implantaten lässt sich ein sicherer Verbund zu Hart- und Weichgewebe herstellen.

### Experimentelle Herstellung von Implantaten:

### Beispiel 1

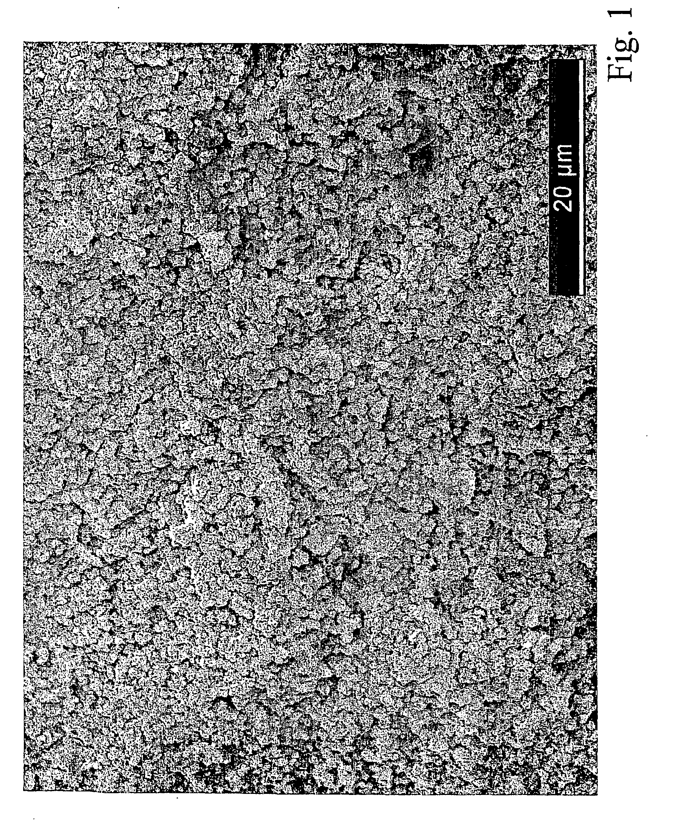

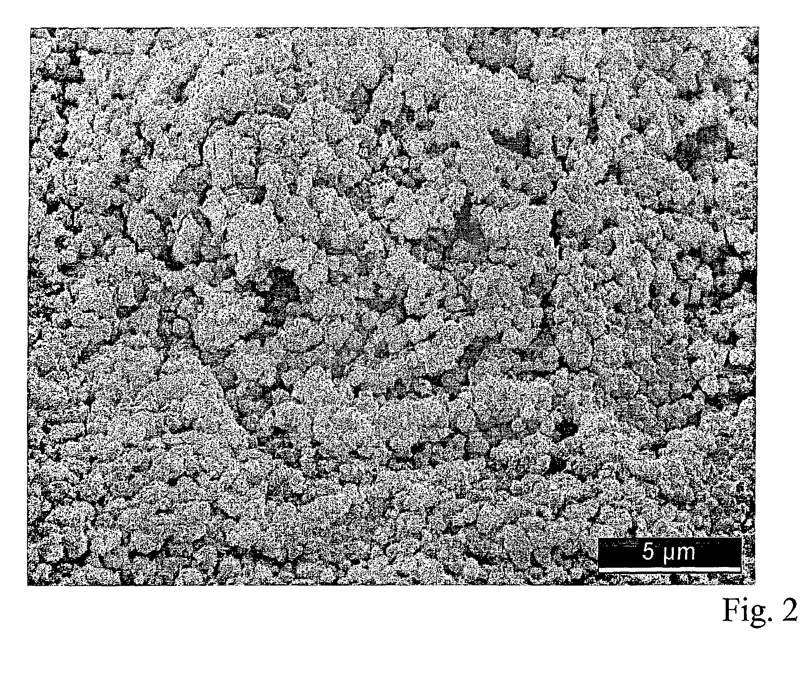

Eine gängige Form, eines Zahnimplantats in Form einer Schraube von 3.5 mm Durchmesser und 10 mm Länge wurde aus Zirkonoxid hergestellt. Die Rohform wurde aus einem zylindrischen keramischen Rohling in an sich bekannter Weise der mechanischen Keramikbearbeitung, hautsächlich durch Schleifen, erhalten. Die in den Knochen einzusetzende Oberfläche wurde nun mit einer Makrorauhigkeit versehen, indem diese mit einem Korn aus Al₂O₃ der mittleren Korngröße 0.1-0.15 mm bei circa 3 bar sandgestrahlt wurde. Anschliessend wurde die aufgerauhte Oberfläche (Makrorauhigkeit) mit einem Kaliumhydroxid - Natriumhydroxid Gemisch mit einem Verhältnis von KOH : NaOH von 1 : 1 bei einer Temperatur von über 190° C während etwa 30 Stunden behandelt. Nach dem Ätzen wurde das Implantat im Ultraschall mit reinem/deionisiertem Wasser behandelt und danach in deionisiertem Wasser gewaschen und gespült. Die dabei entstehende Oberfläche besitzt eine Topographie wie in den Abbildungen 1 und 2 (SEM) gezeigt wird. In den Abbildungen ist erkennbar, dass sich sowohl eine definierte Makrorauhigkeit als auch eine definierte Mikrorauhigkeit einstellt, was für die Osseointegration hervorragend ist.

### Beispiel 2

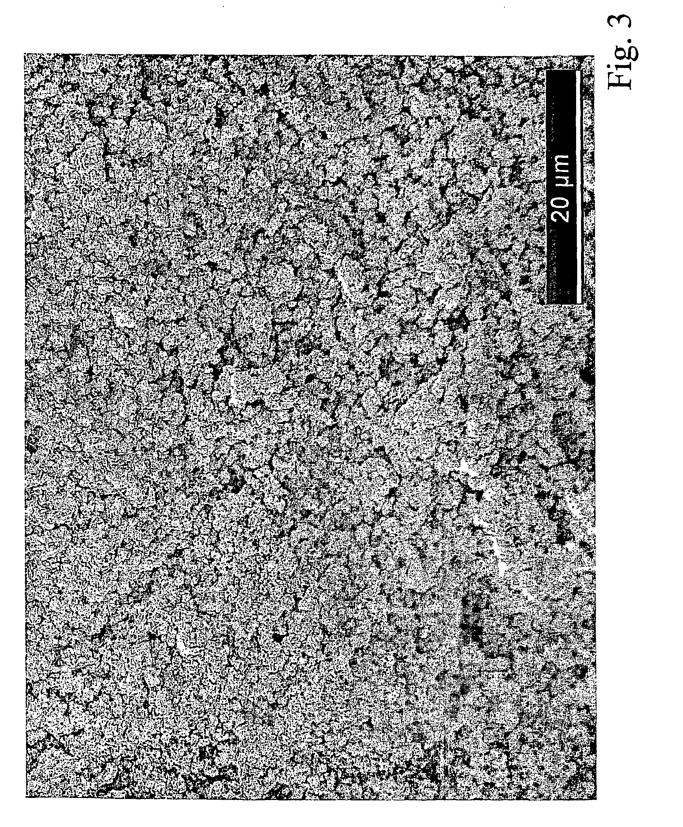

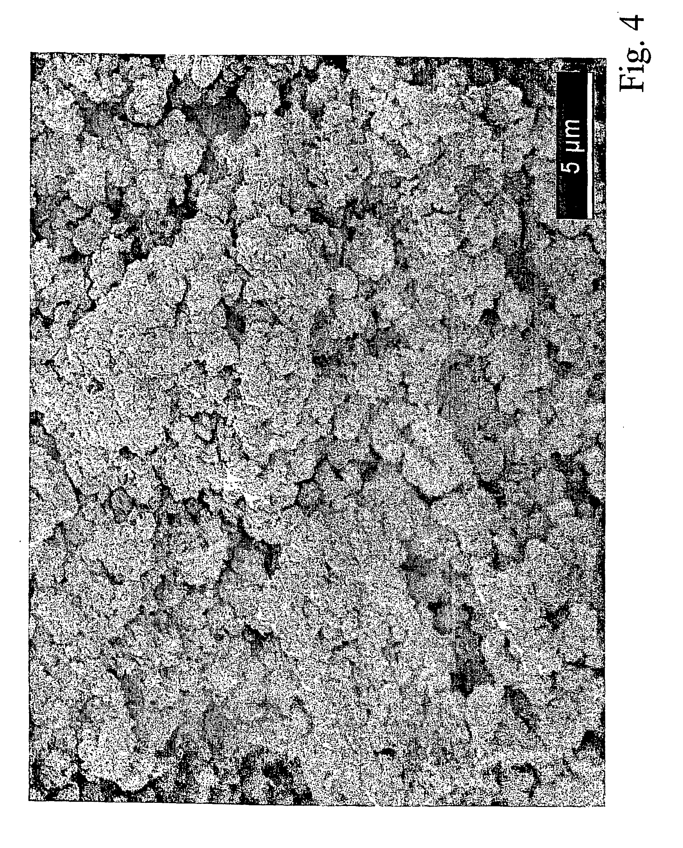

Eine gängige Form eines Zahnimplantats in Form einer Schraube von 3.5 mm Durchmesser und 10 mm Länge wurde aus Zirkonoxid hergestellt. Die Rohform wurde aus einem zylindrischen keramischen Rohling in an sich bekannter Weise der mechanischen Keramikbearbeitung, hauptsächlich durch Schleifen, erhalten. Die in den Knochen einzusetzende Oberfläche wurde nun mit einer Makrorauhigkeit versehen, indem diese mit einem Korn aus Al₂O₃ der mittleren Komgrösse 0.1-0.15 mm sandgestrahlt wurde. Anschliessend wurde die aufgerauhte Oberfläche (Makrorauhigkeit) mit einem Kaliumhydroxid - Natriumhydroxid - Lithiumhydroxid Gemisch mit einem Verhältnis von KOH : NaOH :LiOH von 14 : 6 : 1 bei einer Temperatur von über 190° C während etwa 30 Stunden behandelt. Nach dem Ätzen wurde das Implantat im Ultraschall mit reinem/deionisiertem Wasser behandelt und in deionisiertem Wasser gewaschen und gespült. Die dabei entstehende Oberfläche besitzt eine Topographie wie in den Abbildungen 3 und 4 (SEM) gezeigt wird. In den Abbildungen ist auch hier erkennbar, dass sich sowohl eine definierte Makrorauhigkeit als auch eine definierte Mikrorauhigkeit einstellt, was für die Osseointegration geeignet ist.

### Beispiel 3

Eine gängige Form eines Zahnimplantats in Form einer Schraube von 3.5 mm Durchmesser und 10 mm Länge wurde aus Zirkonoxid hergestellt und wie in Beispiel 2 oberflächenbehandelt. Abweichend von Beispiel 2 wurde nicht nur die in den Knochen, sondern auch die in das Weichgewebe einzusetzende Oberfläche mit einer Rauhigkeit versehen.

### Beispiel 4

Eine gängige Form eines Keramikimplantates in Form einer Hüflpfanne in der Grösse 44 wurde aus Zirkonoxid hergestellt. Die Rohform wurde über einen Grünkörper hergestellt, und dieser keramische Rohling in an sich bekannter Weise bis zum Fertigprodukt gesintert und bearbeitet. Die in den Knochen zu implantierende Oberfläche wurde nun mit einer Makrorauhigkeit versehen, indem diese mit einem Korn der mittleren Korngrösse 0.1-0.15 mm sandgestrahlt wurde. Anschliessend wurde die aufgerauhte Oberfläche (Makrorauhigkeit) mit einem Kaliumhydroxid - Natriumhydroxid Gemisch mit einem Verhältnis von KOH : NaOH von 7 : 5 bei einer Temperatur von über 190° C während etwa 30 Stunden behandelt. Nach dem Ätzen wurde das Implantat im Ultraschall mit reinem/deionisiertem Wasser behandelt und in deionisiertem Wasser gewaschen und gespült.

### Beispiel 5

Eine gängige Form eines Keramikimplantates in Form einer Hüftpfanne in der Grösse 44 wurde aus Zirkonoxid hergestellt. Die Rohform wurde über einen Grünkörper hergestellt, und dieser keramische Rohling in an sich bekannter Weise bis zum Fertigprodukt bearbeitet. Die in den Knochen einzusetzende Oberfläche wurde nun mit einer Makrorauhigkeit versehen, indem diese mit einem Korn der mittleren Komgrösse 0.1-0.15 mm sandgestrahlt wurde. Anschliessend wurde die aufgerauhte Oberfläche (Makrorauhigkeit) mit einem Kaliumhydroxid - Natriumhydroxid - Lithiumhydroxid Gemisch mit einem Verhältnis von KOH: NaOH :LiOH von 14 : 6 : 1 bei einer Temperatur von über 190° C während etwa 30 Stunden behandelt. Nach dem Ätzen wurde das Implantat im Ultraschall mit reinem/deionisiertem Wasser behandelt und in deionisiertem Wasser gewaschen und gespült.

### In vivo-Tests:

Es zeigte sich bei sämtlichen beispielhaft hergestellten Implantaten, dass die Osseointegration resp. die Osteointegration gut erfolgte. Weiterhin zeigt sich auch eine gute Integration an Weichgewebe (z. B. Zahnfleisch).

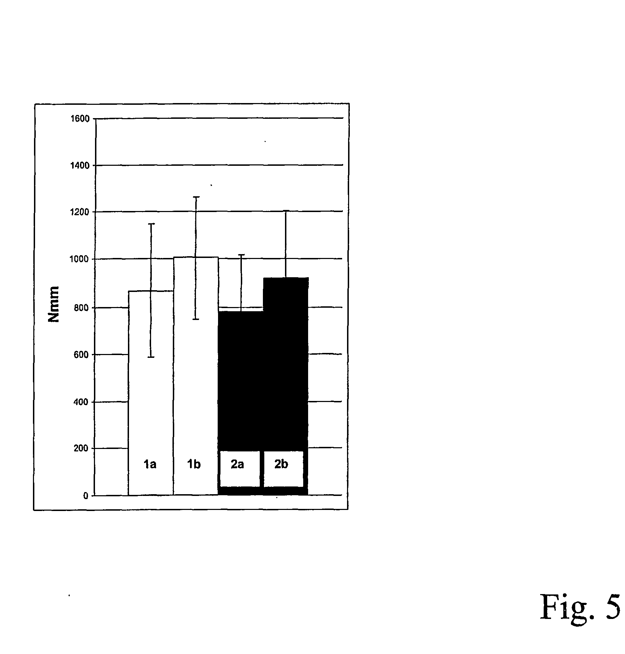

Die Abbildung 5 zeigt entsprechende Resultate von mit zwei verschiedenen Material- und Oberflächenimplantattypen durchgeführten Versuchen. Dabei wurde ein Keramikimplantat mit einem Durchmesser von 4.2 mm und einer Länge von 8 mm mit einer erfindungsgemäss hergestellten Oberfläche im wesentlichen gemäss dem oben beschriebenen Beispiel 1 (Messung 1 in Figur 5) und ein entsprechend dimensioniertes Dentalimplantat mit einer plasmachemisch anodisch oxidierten Oberfläche (Messung 2 in Figur 5) eines Titanimplantates verglichen. Die plasmachemisch anodisch oxidierte Oberfläche entspricht der Oberfläche von kommerziell sehr verbreiteten und häufig verwendeten Implantaten.

Die Implantate wurden in die Beckenschaufel von Schafen implantiert. Nach einer Einheilzeit von 4 (Messung a) und 8 (Messung b) Wochen wurde das Ausdrehmoment bestimmt, welches erforderlich war, um die eingewachsenen Implantate vom Knochen zu lösen.

Wie Abbildung 5 zeigt, findet man ein besseres Einwachsen des neuen Implantates.

## Patentansprüche

1. Implantat mit einer strukturierten, porösen Oberfläche zum wenigstens teilweisen Einsetzen in Hartgewebe wie in einen Knochen und/oder in Weichgewebe, wobei das Implantat keramisch ist,
**dadurch gekennzeichnet, dass**
die poröse Oberfläche wenigstens bereichsweise salzschmelzenmodifiziert ist, wobei das keramische Implantat, gegebenenfalls nach vorgängiger abtragender Oberflächenmodifikation, wenigstens in den Knochen und/oder Weichgewebe ausgesetzten Bereichen in einer Salzschmelze aus Alkali- und/oder Erdalkali-Hydroxiden, oder einer Mischung dieser Salze, oberflächenmodifiziert ist.

2. Implantat nach Anspruch 1, **dadurch gekennzeichnet, dass** das Implantat an der Oberfläche durch eine Salzschmelze durch Ätzung strukturiert ist, wobei insbesondere bei der Ätzung in der Salzschmelze im wesentlichen ausschliesslich eine Abtragung von Material erfolgt ist.

3. Implantat nach Anspruch 1 oder 2, **dadurch gekennzeichnet, dass** dieses wenigstens an der Oberfläche oder bevorzugt vollständig aus Keramik besteht.

4. Implantat nach Anspruch 3, **dadurch gekennzeichnet, dass** dieses Zirkonoxid enthält, welches gegebenenfalls zusätzlich mit Ytriumoxid und Hafniumoxid, versetzt ist, und/oder dass dieses Aluminiumoxid enthält, welches gegebenenfalls zusätzlich mit Siliziumdioxid, Eisen(III)oxid und/oder Natriumoxid versetzt ist, und/oder dass dieses Siliziumnitrid enthält, welches gegebenenfalls zusätzlich mit Siliziumdioxid, Eisen(III)oxid und/oder Natriumoxid versetzt ist, und/oder dass dieses Titanoxid enthält und/oder dass es aus Mischungen aus den genannten Materialien gebildet ist.

5. Implantat nach einem der Ansprüche 3 oder 4, **dadurch gekennzeichnet, dass** es sich um ein Dentalimplantat handelt, dessen im implantierten Zustand dem Knochen und/oder Weichgewebe ausgesetzte Oberfläche salzschmelzenmodifiziert ist.

6. Implantat nach einem der Ansprüche 2 bis 5, **dadurch gekennzeichnet, dass** die poröse Oberfläche wenigstens bereichsweise makrorau, insbesondere sandstrahlmodifiziert ist.

7. Verfahren zur Herstellung eines Implantates nach einem der vorhergehenden Ansprüche, **dadurch gekennzeichnet, dass** das Implantat aus Keramik, gegebenenfalls nach vörgangiger abtragender Oberflächenmodifikation, wenigstens in den Knochen und/oder Weichgewebe ausgesetzten Bereichen in einer Solzschmelze Oberflächenmodifiziert wird, wobei es sich bei der Salzschmelze um eine Salzschmelzen ausschliesslich bestehend aus einem oder mehreren Alkali- und/oder Erdalkali-Hydroxiden handelt.

8. Verfahren nach Anspruch 7, **dadurch gekennzeichnet, dass** es sich bei der Salzschmelze um eine Salzschmelze aus Kaliumhydroxid und/oder Natriumhydroxid und/oder Lithiumhydroxid handelt.

9. Verfahren nach Anspruch 8, **dadurch gekennzeichnet, dass** es sich um eine binäre Salzschmelze aus Kaliumhydroxid und Natriumhydroxid in einem Verhältnis von 2 : 1 - 0.5 : 1, vorzugsweise im Bereich von 1.5 : 1 - 0.75 : 1, insbesondere bevorzugt im Bereich von 1:1 oder 7:5, wobei bevorzugtermassen bei einer Temperatur im Bereich von 100 - 600 °C, insbesondere im Bereich von 150 - 250 °C gearbeitet wird.

10. Verfahren nach Anspruch 8, **dadurch gekennzeichnet, dass** es sich um eine ternäre Salzschmelze aus Kaliumhydroxid, Natriumhydroxid und Lithiumhydroxid in einem Verhältnis im Bereich von 10-20 : 4-10 : 0.5-2, insbesondere im Bereich von 14: 6: 1 handelt, wobei bevorzugtermassen bei einer Temperatur von 150 - 400°C gearbeitet wird.

11. Verfahren nach einem der vorhergehenden Ansprüche 7-10, **dadurch gekennzeichnet, dass** eine Salzschmelze bei einer Temperatur im Bereich von 150°C - 600°C verwendet wird.

12. Verfahren nach einem der vorhergehenden Ansprüche 7-11, **dadurch gekennzeichnet, dass** die Oberfläche wenigstens bereichsweise über eine Dauer von 10 Minuten bis 300 Stunden, bevorzugt von wenigstens 2 Stunden, vorzugsweise von 10 bis 100 Stunden, insbesondere von 25 bis 35 Stunden einer Salzschmelze ausgesetzt wird.

13. Verfahren nach einem der vorhergehenden Ansprüche 7-12, **dadurch gekennzeichnet, dass** die Implantatoberfläche zumindest teilweise nach der Behandlung mit der Salzschmelze gereinigt wird, vorzugsweise im Ultraschall mit deionisiertem Wasser behandelt wird und nachfolgend in deionisiertem Wasser gewaschen und gespült wird.

14. Verfahren nach einem der vorhergehenden Ansprüche 7-13, **dadurch gekennzeichnet, dass** die abtragende Oberflächenmodifikation durch eine Strahlbehandlung erfolgt, insbesondere durch Sandstrahlung, vorzugsweise unter Verwendung von Aluminiumoxid-Partikeln mit einer durchschnittlichen Komgrösse von 0.05 - 0.25 mm oder 0.25 - 0.5 mm, insbesondere bevorzugt mit einem Druck zwischen 1 - 10 bar, vorzugsweise zwischen 1 - 6 bar, insbesondere bevorzugt zwischen 2 - 5 bar.

## Claims

1. An implant with a structured porous surface, for at least partial insertion into hard tissue, for example into a bone, and/or into soft tissue, said implant being ceramic, **characterized in that** the porous surface is modified at least in some regions in a salt melt, wherein the ceramic implant, if applicable after antecedent eroding surface modification, at least in the bones and/or soft tissue exposed areas is surface modified in a salt melt made by alkali and/or earth alkali hydroxides, or a mixture of these salts.

2. The implant as claimed in claim 1, **characterized in that** the implant is structured on the surface by etching in a salt melt, the etching in the salt melt particularly involving essentially only a removal of material.

3. The implant as claimed in claim 1 or 2, **characterized in that** it is made of ceramic at least on the surface or is preferably made entirely of ceramic.

4. The implant as claimed in claim 3, **characterized in that** it contains zirconium oxide, with optional addition of yttrium oxide and hafnium oxide, and/or **in that** it contains aluminum oxide, with optional addition of silicon dioxide, iron(III) oxide and/or sodium oxide, and/or **in that** it contains silicon nitride, with optional addition of silicon dioxide, iron(III) oxide and/or sodium oxide, and/or **in that** it contains titanium oxide, and/or **in that** it is formed from mixtures of said materials.

5. The implant as claimed in one of claims 3 and 4, **characterized in that** it is a dental implant whose surface exposed to the bone and/or soft tissue in the implanted state is modified in a salt melt.

6. The implant as claimed in one of claims 2 through 5, **characterized in that** the porous surface is modified at least in some regions with a macro-roughness, in particular modified by sandblasting.

7. The method for the production of an implant as claimed in one of the preceding claims, **characterized in that** the ceramic implant is surface modified, after antecedent abrasive suface modification if applicable, at least in the bones and/or soft tissue exposed areas in a salt melt, wherein the salt melt is a salt melt exclusively composed of one or more alkali and/or alkaline earth hydroxides.

8. The method as claimed in claim 7, **characterized in that** the salt melt is a salt melt composed of potassium hydroxide and/or sodium hydroxide and/or lithium hydroxide.

9. The method as claimed in claim 8, **characterized in that** the salt melt is a binary salt melt composed of potassium hydroxide and sodium hydroxide in a ratio of 2:1 - 0.5:1, preferably in the range of 1.5:1 - 0.75:1, particularly preferably in the range of 1:1 or 7:5, the work preferably being done at a temperature in the range of 100 - 600°C, particularly in the range of 150 - 250°C.

10. The method as claimed in claim 8, **characterized in that** the salt melt is a ternary salt melt composed of potassium hydroxide, sodium hydroxide and lithium hydroxide in a ratio in the range of 10-20 : 4-10 : 0.5-2, particularly in the range of 14 : 6 : 1, the work preferably being done at a temperature of 150 - 400°C.

11. The method as claimed in one of the preceding claims 7-10, **characterized in that** a salt melt is used at a temperature in the range of 150°C - 600°C.

12. The method as claimed in one of the preceding claims 7-11, **characterized in that** at least some regions of the surface are exposed to a salt melt for a period of 10 minutes to 300 hours, preferably at least 2 hours, preferably 10 to 100 hours, in particular 25 to 35 hours.

13. The method as claimed in one of the preceding claims 7-12, **characterized in that** the implant surface is at least partially cleaned after the treatment with the salt melt, preferably treated by ultrasound with deionized water and then washed in deionized water and rinsed.

14. The method as claimed in one of the preceding claims 7-13, **characterized in that** the surface modification involving removal of material is effected by blasting treatment, in particular by sandblasting, preferably using aluminum oxide particles with an average particle size of 0.05 - 0.25 mm or 0.25 -0.5 mm, particularly preferably with a pressure of between 1 and 10 bar, preferably of between 1 and 6 bar, particularly preferably of between 2 and 5 bar.

## Revendications

1. Implant présentant une surface structurée, poreuse pour une insertion au moins partielle dans un tissu dur, comme dans un os, et/ou dans un tissu mou, l'implant étant en céramique, **caractérisé en ce que** la surface poreuse est modifiée au moins par zones par un sel fondu, l'implant en céramique étant modifié en surface, le cas échéant après une modification de surface préalable par érosion, au moins dans les zones exposées à l'os et/ou au tissu mou, dans un sel fondu d'hydroxydes de métal alcalin et/ou alcalino-terreux ou un mélange de ces sels.

2. Implant selon la revendication 1, **caractérisé en ce que** l'implant est structuré en surface par décapage par un sel fondu, une érosion du matériau ayant en particulier lieu de manière sensiblement exclusive lors du décapage dans le sel fondu.

3. Implant selon la revendication 1 ou 2, **caractérisé en ce qu'**il est constitué au moins en surface ou de préférence complètement de céramique.

4. Implant selon la revendication 3, **caractérisé en ce qu'**il contient de l'oxyde de zirconium, qui est le cas échéant additionné en plus d'oxyde d'yttrium et d'oxyde d'hafnium et/ou **en ce qu'**il contient de l'oxyde d'aluminium qui est le cas échéant additionné en plus de dioxyde de silicium, d'oxyde de fer (III) et/ou d'oxyde de sodium et/ou **en ce qu'**il contient du nitrure de silicium, qui est le cas échéant additionné en plus de dioxyde de silicium, d'oxyde de fer (III) et/ou d'oxyde de sodium et/ou **en ce qu'**il contient de l'oxyde de titane et/ou **en ce qu'**il est formé à partir de mélanges des matériaux mentionnés.

5. Implant selon l'une quelconque des revendications 3 ou 4, **caractérisé en ce qu'**il s'agit d'un implant dentaire, dont la surface exposée à l'os et/ou au tissu mou à l'état implanté est modifiée par un sel fondu.

6. Implant selon l'une quelconque des revendications 2 à 5, **caractérisé en ce que** la surface poreuse est modifiée au moins par zones par une macrorugosité, en particulier sablée.

7. Procédé pour la réalisation d'un implant selon l'une quelconque des revendications précédentes, **caractérisé en ce que** l'implant en céramique, le cas échéant après une modification de surface par érosion préalable, est modifié en surface au moins dans les zones exposées à l'os et/ou au tissu mou, dans un sel fondu, où il s'agit, pour le sel fondu, d'un sel fondu constitué exclusivement d'un ou de plusieurs hydroxydes de métal alcalin et/ou alcalino-terreux.

8. Procédé selon la revendication 7, **caractérisé en ce qu'**il s'agit, pour le sel fondu, d'un sel fondu d'hydroxyde de potassium et/ou d'hydroxyde de sodium et/ou d'hydroxyde de lithium.

9. Procédé selon la revendication 8, **caractérisé en ce qu'**il s'agit d'un sel fondu binaire d'hydroxyde de potassium et d'hydroxyde de sodium dans un rapport de 2:1-0,5:1, de préférence dans la plage de 1,5:1-0,75:1, en particulier de préférence dans la plage de 1:1 ou 7:5, en travaillant de préférence à une température dans la plage de 100-600°C, en particulier dans la plage de 150-250°C.

10. Procédé selon la revendication 8, **caractérisé en ce qu'**il s'agit d'un sel fondu ternaire d'hydroxyde de potassium, d'hydroxyde de sodium et d'hydroxyde de lithium dans un rapport dans la plage de 10-20:4-10:0,5-2, en particulier dans la plage de 14:6:1, en travaillant de préférence à une température de 150-400°C.

11. Procédé selon l'une quelconque des revendications précédentes 7 à 10, **caractérisé en ce qu'**on utilise un sel fondu à une température dans la plage de 150°C-600°C.

12. Procédé selon l'une quelconque des revendications précédentes 7 à 11, **caractérisé en ce que** la surface est exposée à un sel fondu, au moins par zones, pendant une durée de 10 minutes à 300 heures, de préférence d'au moins 2 heures, de préférence de 10 à 100 heures, en particulier de 25 à 35 heures.

13. Procédé selon l'une quelconque des revendications précédentes 7 à 12, **caractérisé en ce que** la surface de l'implant est nettoyée au moins partiellement après le traitement au sel fondu, de préférence traitée dans des ultrasons avec de l'eau désionisée et ensuite lavée et rincée dans de l'eau désionisée.

14. Procédé selon l'une quelconque des revendications précédentes 7 à 13, **caractérisé en ce que** la modification de surface par érosion a lieu par un traitement aux jets, en particulier par sablage, de préférence en utilisant des particules d'oxyde d'aluminium présentant une grosseur moyenne des grains de 0,05-0,25 mm ou de 0,25-0,5 mm, en particulier de préférence à une pression entre 1-10 bars, de préférence entre 1-6 bars, en particulier de préférence entre 2-5 bars.
